# EUROPEAN PATENT SPECIFICATION

(11) **EP 0 275 300 B1**
(45) Date of publication and mention of the grant of the patent: **03.01.1996**
(21) Application number: 87905142.3
(22) Date of filing: 31.07.1987
(51) Int. Cl.: C12N 15/85

(54) **RECOMBINANT VACCINE**
REKOMBINANTER IMPFSTOFF
VACCIN RECOMBINANT

(30) Priority: 01.08.1986 AU 7212/86
(43) Date of publication of application: 27.07.1988
(73) Proprietor: COMMONWEALTH SCIENTIFIC AND INDUSTRIAL RESEARCH ORGANISATION, Campbell, Australian Capital Territory 2601 (AU); THE AUSTRALIAN NATIONAL UNIVERSITY, Acton, Australian Capital Territory 2601 (AU)
(72) Inventor: RAMSHAW, Ian, Allister, Duffy, ACT 2611 (AU); BOYLE, David, Bernard, Geelong, VIC 3220 (AU); COUPAR, Barbara, Elizabeth, Howieson, East Geelong, VIC 3219 (AU); ANDREW, Marion, Elizabeth, Belmont, VIC 3216 (AU)
(74) Representative: Collier, Jeremy Austin Grey
(86) International application number: AU8700246
(87) International publication number: WO8800971

(56) References cited:
- EP-A- 0 138 133
- EP-A- 0 176 170
- EP-A- 0 181 117
- EP-A- 0 206 920
- EP-A- 0 206 939
- AU-A- 1 167 483
- AU-A- 3 663 584
- AU-A- 5 945 386
- Proceedings of the National Academy of Science USA, Volume 82, No.3 issued 1985, February (Washington D.C.), R.J. KAUFMAN, "Identification of the Components Necessary for Adenovirus Translational Control and their Utilization in cDNA Expression Vectors", see pages 689-693
- ADV. IMMUNOPHARMACOL. vol 3, Proc. Int. Conf. 3rd, 1985, (publ. 1986), pages 223-230, Ed. Pergamon Press, Oxfored, GB C.A. DINARELLO
- BIOLOGICAL ABSTRACT, RRM no. 29067133 H. KAWAMURA et al.:
- CHEMICAL ABSTRACT, vol. 92, no. 19, May 12, 1980, page 446, abstract 162033q, Columbus, Ohio, US T.A. BEKTEMIROV et al.:
- PROC. NATL. ACAD. SCI. USA; vol 83, no. 16, Ausgust, 1986, pages 5894-5898, Washington, US T. YOKOTA et al.:
- NATURE, vol. 320, no. 6062, April 1986, London, GB; pages 537-540 S.-L. HU et al.
- THE YEARBOOK OF AGRICULTURE, US Department of Agriculture, 1986, pages 104-108 K.J. CREMER
- BIOLOGICAL ABSTRACTS, vol. 79, 1985, no. 13273 P.J. GROB et al.
- J. IMMUNOL., vol. 130, no. 5, May 1983, pages 2191-2194 M.J. STARUCH et al.
- NATURE, vol. 329, no. 6139, 8-14th October 1987, London, GB pages 545-546 I.A. RAMSHAW et al.

## Description

This invention concerns new vaccines developed using recombinant DNA technology to provide useful immune responses in circumstances where traditional vaccines may not be sufficiently effective.

Many existing live or killed vaccines are not without disadvantages, often significant, in respect of, for example, high production costs, poor response, low response to poorly immunogenic antigens, instability and a requirement for adjuvants. Furthermore, alternative vaccine preparations based on agents such as purified proteins or synthetic peptide antigens frequently offer only poor protection. In response to these problems, attention has turned to the development of vaccines in which recombinant DNA methods have been used to introduce antigens to which immunity is required, into carrier viruses such as vaccinia.

The advantage of the recombinant DNA approach is that an infectious recombinant virus simultaneously synthesizes the foreign polypeptide and viral antigen, which can then be delivered to a host immune system as a superficial skin lesion. Vaccinia viruses have, for example, been modified for expression of the genes for hepatitis B, human immunodeficiency virus, influenza and malaria antigens; the construction of recombinant viruses carrying other antigens of medical or veterinary importance is under investigation.

In some instances, however, the immune response of recombinant vaccines may be of limited nature and magnitude. Thus, while peripheral immunization with vaccinia-influenza recombinants provides good protection against lower respiratory tract infection, it fails to induce immunity in the upper respiratory tract. On the other hand, peripheral immunization with recombinant vaccines may prove ineffective when local rather than systemic immunity is required, as in say the gastro-intestinal tract.

There have been various attempts to remedy these deficiencies, including expression of vaccine antigens through viruses having stronger promoters, such as poxvirus, but to date these have not met with significant success. The present invention provides an effective means for enhancing the immune response to the specific foreign antigenic polypeptides of recombinant vaccines.

The immune system is regulated in part by molecules, known as lymphokines, which are released by lymphocytes and help or modify the functions of other classes of lymphocytes. The present invention is based on a recognition that the expression of appropriate lymphokines from recombinant bacterial or viral vaccines can boost and/or modify the immune response to viral, bacterial or co-expressed foreign antigenic polypeptides.

Adv. Immunopharmacol 3, Proc.Int.Conf. 3rd 1985 pages 223-230, Editor: L. Chedid, Pergamon, Oxford, UK (published 1986) C.A. Dinarello "The Biological Activities of Human Interleukin-1: Purified and Recombinant Materials" proposes that a fused IL-1/peptide could be produced in vitro using a bacterium.

According to one aspect of the present invention, there is provided a recombinant vaccine comprising an animal cell vector which incorporates a first nucleotide sequence capable of being expressed as all or an immunogenic part of an antigenic polypeptide, which is foreign to the host vector and is not a lymphokine, together with a second nucleotide sequence capable of being expressed as all or a lymphokine-effective part of a lymphokine other than interleukin-2 effective in enhancing the immune response to the antigenic polypeptide. The invention also provides a method for the production of such a recombinant vaccine which method comprises the step of inserting into an animal cell vector a nucleotide sequence capable of being expressed as all or a lymphokine-effective part of a lymphokine other than interleukin-2, and the further step of inserting into the animal cell vector a nucleotide sequence capable of being expressed as all or an immunogenic part of an antigenic polypeptide which is foreign to the host vector and is not a lymphokine.

According to another aspect of the present invention there is provided a recombinant vaccine for use in the treatment of an immunodeficient or immunosuppressed human or animal, which comprises an animal cell vector which incorporates a first nucleotide sequence capable of being expressed as all or an immunogenic part of an antigenic polypeptide, which is not a lymphokine, together with a second nucleotide sequence capable of being expressed as all or a lymphokine-effective part of a lymphokine other than interleukin-2 effective in enhancing the immune response to the antigenic polypeptide.

According to a further aspect of the invention there is provided a recombinant vaccine for use in the treatment of immunodeficient or immunosuppressed individuals infected with the human immunodeficiency virus (HIV), which comprises an animal cell vector which incorporates a nucleotide sequence capable of being expressed as all or an immunogenic part of an antigenic polypeptide derived from the human immunodeficiency virus (HIV), together with a second nucleotide sequence capable of being expressed as all or a lymphokine-effective part of a lymphokine effective in enhancing the immune response of the individual to the HIV antigenic polypeptide.

Furthermore, the invention provides the use of a recombinant vaccine comprising an animal cell vector which incorporates a first nucleotide sequence capable of being expressed as all or an immunogenic part of an antigenic polypeptide, which is not a lymphokine, together with a second nucleotide sequence capable of being expressed as all or a lymphokine-effective part of a lymphokine other than interleukin-2 effective in enhancing the immune response to the antigenic polypeptide, for the manufacture of a medicament for the treatment of an immunodeficient or immunosuppressed human or animal.

When the first nucleotide sequence capable of being expressed as all or an immunogenic part of an antigenic polypeptide is a "native" sequence of the host vector itself, for example vaccinia or herpes virus, administration of the vaccine of this invention provides augmentation and/or selective induction of the immune response to the "native" antigenic polypeptide.

When the animal cell vector incorporates a nuclebtide sequence capable of being expressed as all or an immunogenic part of an antigenic polypeptide which is foreign to the host vector, administration of the vaccine to an individual will result in augmentation and/or selective induction of the immune response of the individual to both antigenic polypeptide of the host vector and to co-expressed foreign antigenic polypeptide.

A recombinant vaccine according to the invention may be used to produce an immune response in a human or animal, in particular an immunodeficient or immunosuppressed human or animal, by a method which comprises the step of administering to the human or animal a recombinant vaccine as broadly described above.

It will be appreciated from the broad description set out above that the present invention has particular application in the augmentation of immune responses in immunodeficient or immunosuppressed individuals. In one particularly important aspect of this invention, there is provided the recombinant vaccine for use in the treatment of immunodeficient or immunosuppressed individuals infected with the human immunodeficiency virus (HIV), which comprises an animal cell vector which incorporates a nucleotide sequence capable of being expressed as all or an immunogenic part of an antigenic polypeptide derived from the human immunodeficiency virus (HIV), together with a second nucleotide sequence capable of being expressed as all or a lymphokine-effective part of a lymphokine effective in enhancing the immune response of the individual to the HIV antigenic polypeptide.

The lymphokines which may be expressed in vaccines according to this invention include as appropriate those designated interleukin-1 (IL-1), interleukin-2 (IL-2), interleukin-3 (IL-3), interleukin-4 (IL-4) and γ-interferon (γ-IFN).

Interleukin-1 is a peptide hormone largely produced by activated macrophages. IL-1 modulates the proliferation, maturation and functional activation of a broad spectrum of cell types (1-3) and plays a major role in the initiation and amplification of immune and inflammatory responses through its action on these diverse cell populations (4). The gene for murine (5) and human (6) IL-1 has been cloned and expressed in E.coli.

Interleukin-2 is a lymphokine produced by helper T cells and is active in controlling the magnitude and type of the immune response (7). Other functions have also been ascribed to IL-2 including the activation of NK cells (8) and the stimulation of cell division in large granular lymphocytes and B cells (9). Numerous studies in mice and humans have demonstrated that deficient immune responsiveness both in vivo and in vitro can be augmented by IL-2. For example, exogenous IL-2 can restore the immune response in cyclophosphamide-induced immunosuppressed mice (10) and athymic (nude) mice (11). Furthermore, IL-2 can restore responsiveness of lymphocytes from patients with various immunodeficiency states such as leprosy and cancer (12). IL-2 has also been used for the treatment of cancer (13). The gene for immune (14) and human (15) IL-2 has been cloned and sequenced.

Interleukin-3 is a hormone-like glycoprotein produced by lectin or antigen activated T lymphocytes and possibly other cells within the bone marrow. The hormone stimulates the growth and differentiation of haematopoietic progenitor cells and multipotential stem cells and has been described under a variety of names, among them multi-colony stimulating factor, and haematopoietic growth factor (16). The gene for mouse IL-3 has been cloned and sequenced (17).

Interleukin-4 is a T cell derived factor that acts as an induction factor on resting B cells, as a B cell differentiation factor and as a B cell growth factor (18). The factor also stimulates T cells and acts as a mast cell growth factor (18). The gene for murine (19) and human (20) IL-4 has been isolated and sequenced.

γ-interferon is also a T cell derived molecule which has profound effects on the immune response. The molecule promotes the production of immunoglobulin by activated B cells stimulated with interleukin-2. γ-interferon also increases the expression of histocompatibility antigens on cells which associate with viral antigens to stimulate cytotoxic T cells. The gene for human γ-interferon has been isolated and sequenced (21).

The co-expression of a lymphokine and an antigenic polypeptide by a recombinant vaccine (such as a recombinant virus vaccine) ensures that they are produced together by the same infected cells in a very localized area. This can be expected to lead to an elevation and acceleration of response to the virus vector component of the vaccine, e.g. vaccinia virus, with attendant benefits such as a reduction in the risks of complication associated with the use of vaccinia virus in normal individuals, and to those unidentifiable individuals who react adversely to vaccinia virus. Also, where there are immunological defects, as in the case of patients suffering from AIDS, leprosy or cytomegalovirus infection, co-expression of lymphokine could be instrumental in overcoming the defects to allow a normal response to the antigenic polypeptide and/or vector virus.

Furthermore, it is anticipated that the present invention will prevent or at least minimise the complications such as generalised vaccinia that can occur when vaccine is administered inadvertently to immunodeficient recipients (32).

Lymphokines (e.g. IL-1, IL-3, IL-4 and γ-IFN), are involved in the control and augmentation of responses in parts of the immune system including granulocyte-macrophage lineage, eosinophil differentation and mucosal immunity. Construction of co-expressive vaccines will enable advantage to be taken of these specific modes of activity. Thus, as they are believed to have a role in the generation of protective responses at mucosal surfaces, such as in the gut, which promote expulsion of and immunity to parasites, a vaccine co-expressing IL-3 (or other lymphokine) with a helminth or other parasite antigenic polypeptide would be expected to give rise to enhanced immunity compared to that from the parasite antigen alone. Whilst a specific example of co-expression of the influenza haemagglutinin (HA) is described in detail herein, it will be appreciated that the present invention may be applied for the co-expression of other antigens including hepatitis virus, herpes simplex virus, Epstein-Barr virus and human immunodeficiency virus antigens, as well as malaria antigens.

As previously described, vaccinia virus has been used as an animal cell vector to deliver antigens of unrelated infectious agents such as hepatitis B virus (22) and human immunodeficiency virus (23). The expression of an inserted gene in vaccinia virus requires that the gene be placed next to a vaccinia promoter. The promoter usually used is designated p7.5 (24). This chimeric gene is then placed next to a DNA fragment of vaccinia virus taken from a non-essential region of the virus. Insertion into infectious virus is by homologous recombination in which a marker rescue is used to select for virus recombinants. By way of example, the marker rescue can be either selection for thymidine kinase negative (TK⁻) virus in which the foreign gene has been inserted and thereby inactivating the TK gene; or by selecting for TK⁺ virus in which the foreign gene is flanked by the herpes simplex TK gene. The latter is generally used to construct double recominants that is, viruses expressing two foreign genes.

The expression of lymphokine genes in vaccinia virus may be detailed as two stages; the first is to create a plasmid in which the lymphokine is under the control of a vaccinia promoter 7.5 and downstream from a thymidine kinase (HSV) gene. This plasmid is then used to transfect TK⁻ cells previously infected with a TK⁻ vaccinia virus expressing another foreign gene. TK⁺ recombinant virus is then selected by culturing cells in the presence of methotrexate.

Although this invention has primarily been described with reference to vaccinia virus as the animal cell vector, it is to be understood that the inventive concept resides in co-expression of an antigenic polypeptide and lymphokine, and this concept may be realized using other animal cell vectors, such as other poxvirus, herpes virus, adenovirus or bacteria.

It is also to be understood that the invention is not limited by application to man or other species specifically mentioned herein, but may find application in a wide range of animal species.

Methods for construction and testing of recombinant vaccines according to this invention will be well known to those skilled in the art, however, for better understanding of the invention some typical techniques will now be described. Standard procedures for endonuclease digestion, ligation and electrophoresis were carried out in accordance with the manufacturer's or supplier's instructions. Standard techniques are. not described in detail and will be well understood by persons skilled in the art.

### EXAMPLE 1

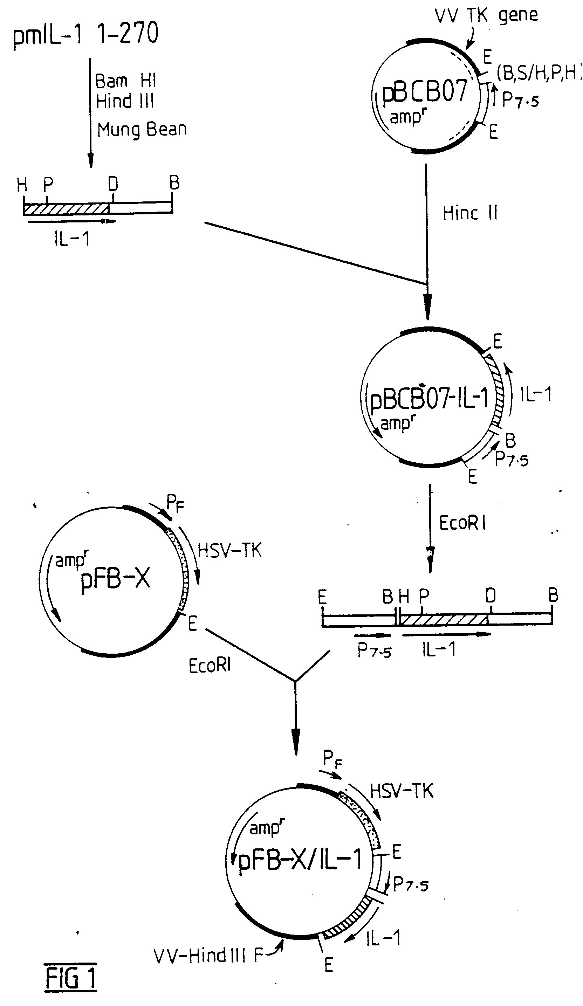

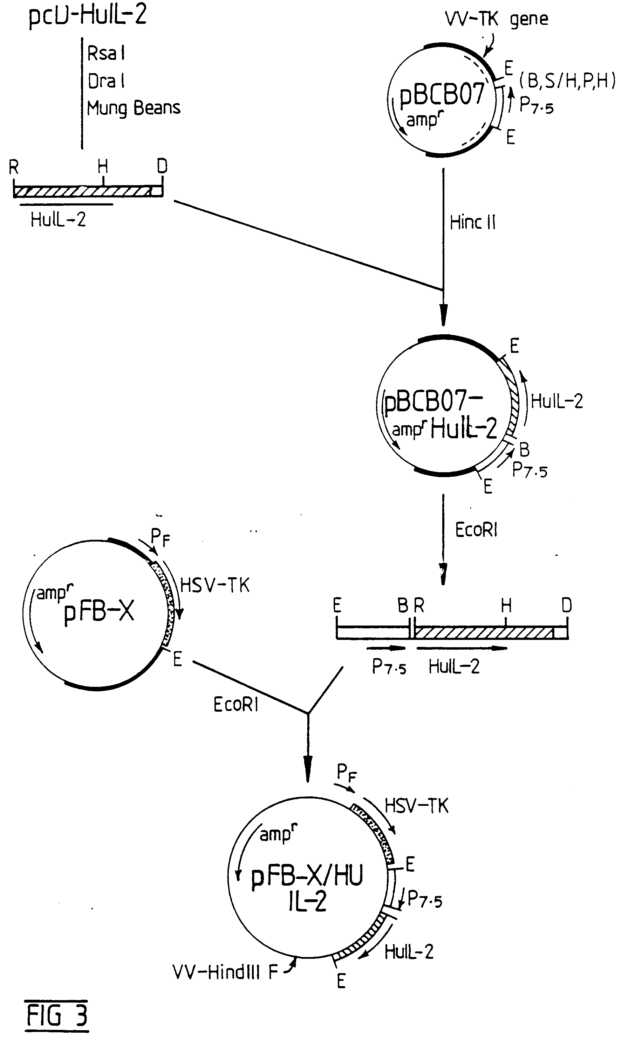

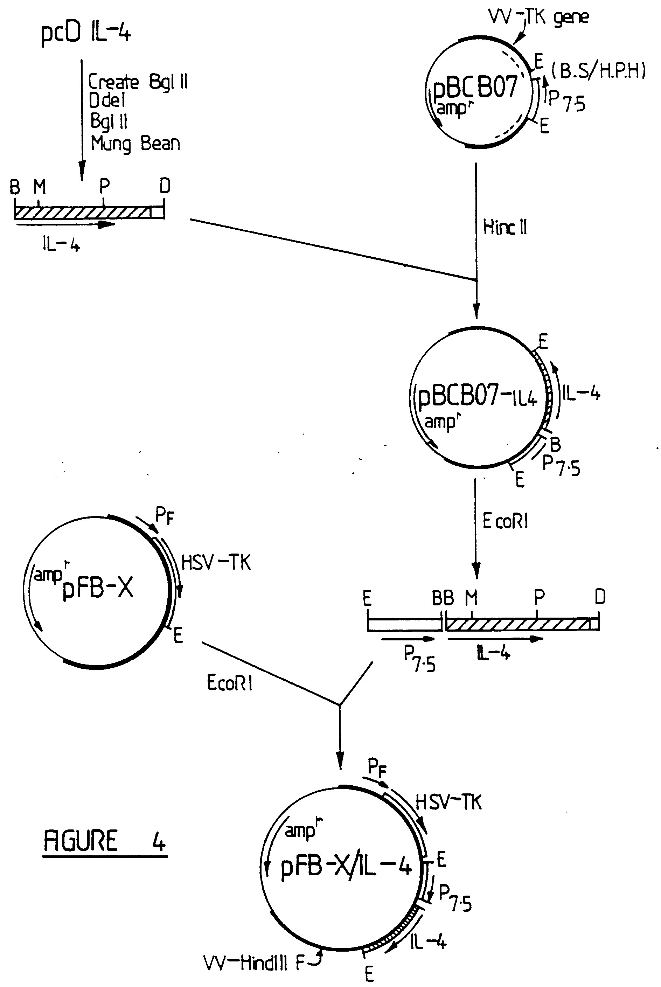

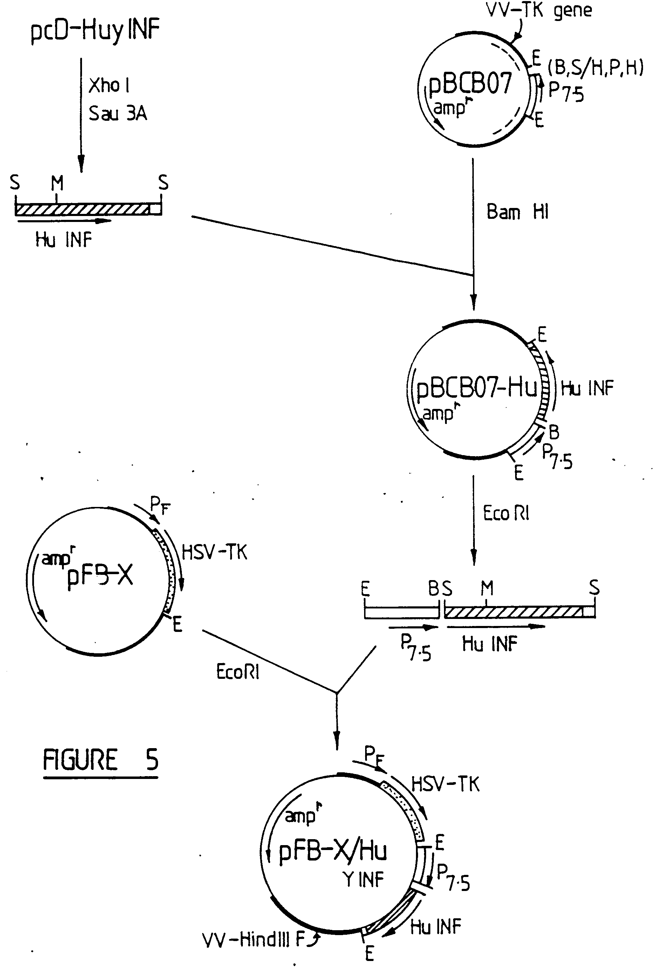

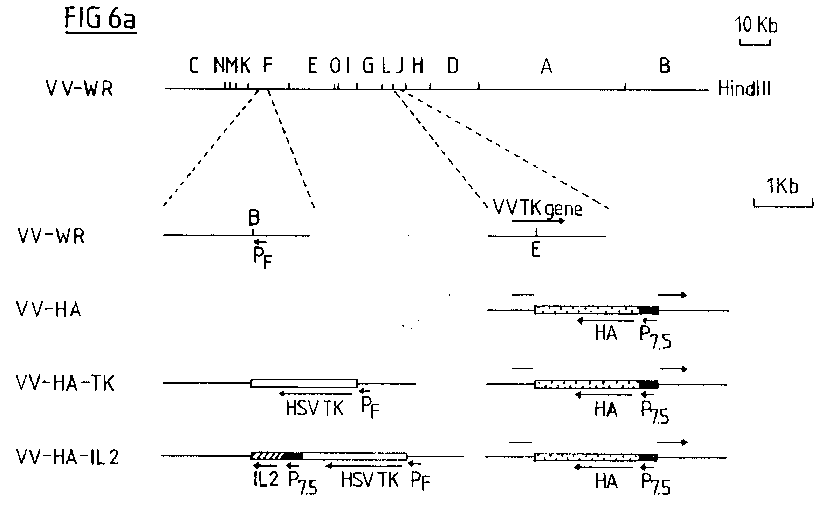

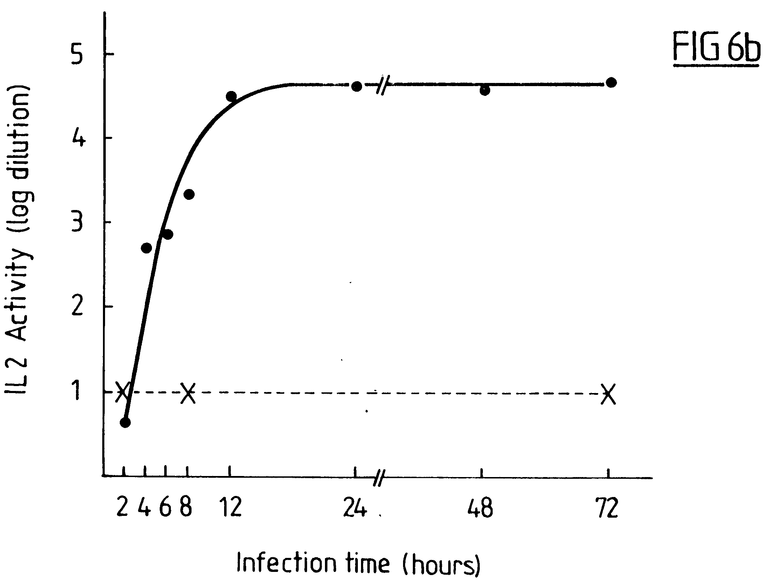

Plasmids containing IL-1, IL-2, IL-3, IL-4 and γ-interferon are shown in Figures 1-5. Plasmids pmIL-11.270, pcD-JL-3, pcD-HuIL-2, pcD-IL-4 and pcD-HuγINF were obtained from DNAX Research Institute. The excised coding sequence is shown as the hatched bar. It is necessary to use different restriction endonucleases to create suitable termini for insertion into plasmid; these are detailed in the respective diagrams. pBCB07 (25) contains the vaccinia 7.5K promoter, pFB-X (27) contains the HSV TK coding sequence (stippled) inserted at a BamHl site downstream from a promoter in the vaccinia HindIII region. The recoiiibinant plasmids pFB-X/IL1, pFB-X/IL2, pFB-X/IL3, pFB-X/IL4, pRB-X/γ-interferon contain HSV-TK and lymphokine genes 3' to different vaccinia virus promoters and with flanking sequences derived from the HindIII F fragment of vaccinia virus. The orientations of genes and promoters are shown with arrows, vaccinia virus sequences with solid lines and plasmid DNA by thin lines. The pFB-plasmids with inserted lymphokine genes are used to transfect 143B (TK⁻) cells previously infected with a TK⁻ vaccinia virus according to conditions previously described (24). The site of insertion of HSV TK and lymphokine coding sequences and transposed vaccinia promotors in the vaccinia virus genome are shown in Figure 6. Vaccinia-virus WR strain HindIII restriction fragments are shown in the top line. Lower lines show in expanded form the DNA configurations at insertion sites in the HindIII J and F fragments. Orientations of coding sequences and promotor sequences are shown with arrows.

### EXAMPLE 2

This example describes the construction of a recombinant vaccinia virus (VV) expressing murine IL2 and the effect of the lymphokine on virus growth and immunogenicity.

### Figure 6 shows:

(a) Genomic configuration of VV recombinants. A HindIII map of VV WR strain is shown with insertion points at EcoRI (E) and BamHI (B) sites in the J and F fragments respectively. Arrows indicate orientations of VV TK gene, VV promoters and inserted influenza HA, HSV TK and murine IL2 coding sequences.
(b) Time course of IL2 production by VV-HA IL2-infected human 143B cells. IL2 activity in VV-HA-IL2-infected supernatants, circles and solid line;p VV-HA or uninfected cell supernatants, crosses and dotted line.

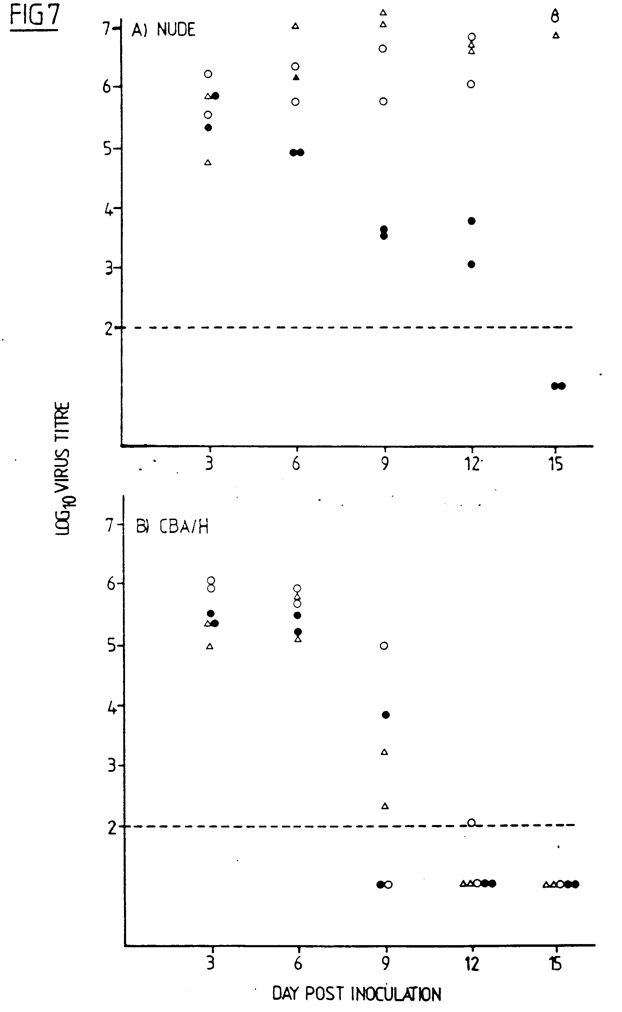

Figure 7 shows the growth of vaccinia virus recombinants in the foot pads of athymic Swiss outbred nude mice (a) and euthymic CBA/H mice (b). 2x10⁷ PFU of VV-HA (triangles), VV-HA-TK (open circles) or VV-HA-IL2 (closed circles) were injected subcutaneously in 20µl into hind foot pads which were assayed for infectious virus on 143B cells on the indicated days. Points represent the titres of infectious virus present in individual mice.

As shown schematically in Figure 6a, cDNA encoding murine IL2 (14) was inserted into the HindIII F region of a VV recombinant, VV-HA (26), which already expressed the influenza haemagglutinin (HA). The IL2 recombinant virus, VV-HA-IL2, coexpressed HA and IL2 using the same VV 7.5Kd promoter but from separate sites in the viral genome. Since the herpes simplex virus (HSV) thymidine kinase (TK) gene was used as a selectable marker for virus construction a control virus VV-HA-TK, expressing HSV TK but not IL2 was constructed. Significant levels of biologically active IL2 were detected in supernatants from human 143B cells infected with VV-HA-IL2 within 4 hours and reached maximum activity around 12 hours (Figure 6b).

Athymic nude mice were inoculated into the right hind footpad with VV-HA-IL2 or control virus (VV-HA-TK). VV-HA-IL2 induced a mild swelling in the foot which resolved after several days; in contrast VV-HA-TK produced a severe necrotic lesion that remained unresolved for 30 days. After this time, high titres of virus (6x10⁵-1.5x10⁷PFU) were recovered from the feet of the VV-HA-TK inoculated mice but not from mice given VV-HA-IL2. This suggested that the IL2 produced by the recombinant virus enabled immunodeficient mice to control the virus infection. The kinetics of viral clearance from the feet of CBA/H mice were not significantly different for VV-HA-TK and VV-HA-IL2 (Figure 7a). However, in nude mice, although titres of both VV-HA-TK and VV-HA-IL2 were high at day 3, indicating comparable rates of replication, VV-HA-IL2 was cleared by day 15 when no virus was detected in the feet. Titres of VV-HA-TK still remained high at day 15 (Figure 7b). Furthermore, when nude mice were injected intravenously (i.v.) with 10⁶ PFU of VV-HA-IL2 or VV-HA-TK, mice given VV-HA-IL2 appeared unaffected by the virus whereas all mice given VV-HA-TK were moribund by day 15. Consistent with this result infectious virus was recovered from spleens and lungs of VV-HA-TK- but not VV-HA-IL2-infected mice (Table 1).

**TABLE 1.**

| Vaccinia Virus recovered from Lungs and Spleen | | | |
|---|---|---|---|
| Mouse Strain | Organ | Log.10 VV-HA-TK | Log.10 VV-HA-IL2 |
| | | | |
| CBA/H | Lung | <2.0 | <2.0 |
| | Spleen | <2.0 | <2.0 |
| | | | |
| Outbred | Lung | 4.91±0.27 | <2.0 |
| Nude | Spleen | 3.64±0.11 | <2.0 |
| | | | |

Lungs and spleens were collected from 3-4 mice/group 11 days post i.v. inoculation with 10⁶ PFU of the indicated virus.

### Methods

(a) Murine IL-2 cDNA was subcloned from pcD-IL-2, (14) generously provided by Dr K.I. Arai, DNAX Research Institute, Palo Alto, California. VV-HA-IL1 and VV-HA-TK were constructed by insertion of the HSV TK gene plus a chimeric promoter-IL2 fragment or alone, into the HindIII F region of the recombinant VV-HA (previously described as VV-PR8-HA6) (26) using plasmids as described in Example 1 and appropriate selection protocols (27, 28).
(b) 143B cells 2x10⁶ were infected with VV-HA-IL2 at 5 PFU/cell. The supernatants (1.5ml), harvested at the time points indicated, were assayed for IL2 activity using CTLL-2 cells (29) and the colourimetric method for cell growth of Mosmann (30). The results are presented as the log dilution of supernate producing 50% of maximum proliferation in the cell cultures.

### EXAMPLE 3

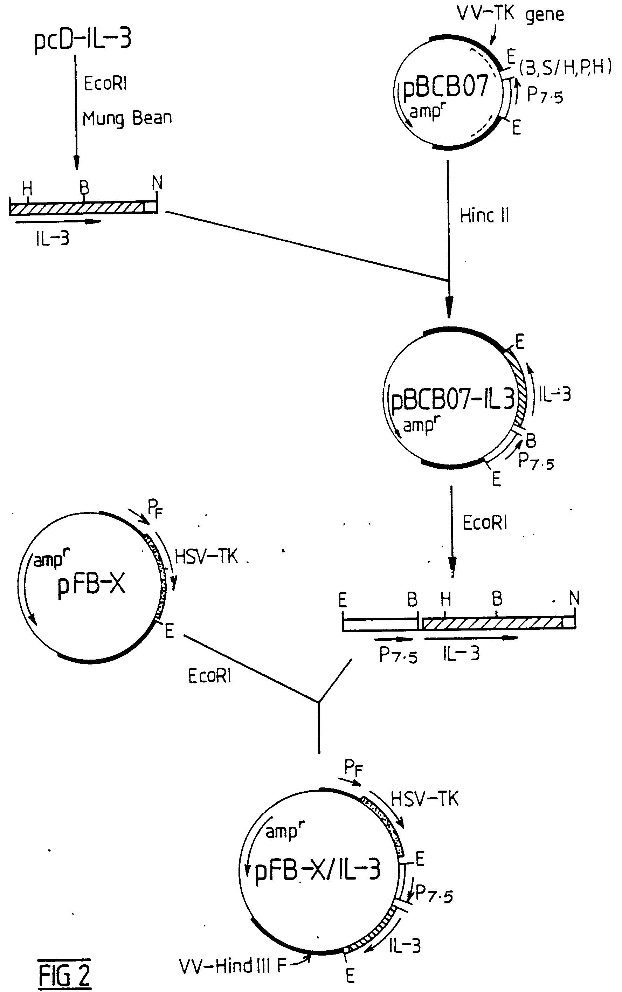

A vaccinia virus expressing IL-3 was constructed as shown in Figure 2, using the methods described in Example 1.

Irradiated mice (650 Rads) injected with vaccinia virus (10⁶PFU intravenously) expressing IL-3 (VV-IL-3) show a reconstituted haemotoporetic system within seven days. Spleen cell counts are given below:

| Spleen Cell Number | | |
|---|---|---|
| | VV-IL-3 | NIL |
| 4 days | 3 x 10⁷ | 5 x 10⁶ |
| 7 days | 2 x 10⁸ | 2 x 10⁶ |
| 10 days | 1 x 10⁸ | 3 x 10⁶ |

In addition, the injection of vaccinia virus expressing IL-3 (VV-IL-3) protects mice against the lethal effects of irradiation, as follows:

| | Deaths | |
|---|---|---|
| Irradiation Dose | VV-IL-3 | NIL |
| 950 Rads | 0/6 | 6/6 |

### EXAMPLE 4

This example describes the construction of a recombinant adenovirus expressing interleukin genes.

The starting virus for the adenovirus construct is adenovirus type 5 deletion mutant dl 327 that lacks the Xba fragment from 78.5 map units to 84.7 map units in early region 3 (31). This deletion mutant allows the insertion of DNA without exceeding the amount of DNA that can be included in the virus particle. The removal of the E3 region also prevents production of a virus protein that complexes with the major histocompatibility heavy chain protein and reduces the cell-mediated immune response to the virus. The Bam fragment from 60 map units to the right hand end of the viral DNA is cloned in plasmid. The plasmid DNA is cut downstream of the E3 promoter with a suitable restriction enzyme and the interleukin gene inserted in place of the original E3 gene, under the control of the natural E3 promoter. The resulting plasmid containing the interleukin gene in the 60 to 100 map unit fragment of dl 327 is cut with the appropriate restriction enzyme to separate viral and plasmid DNA and transfected into cells together with the overlapping EcoRl A fragment (0 to 76 map units) of wild type virus. Recombination between the two overlapping DNA fragments will reconstitute viable adenovirus in which the E3 gene is replaced by the interleukin gene.

### EXAMPLE 5

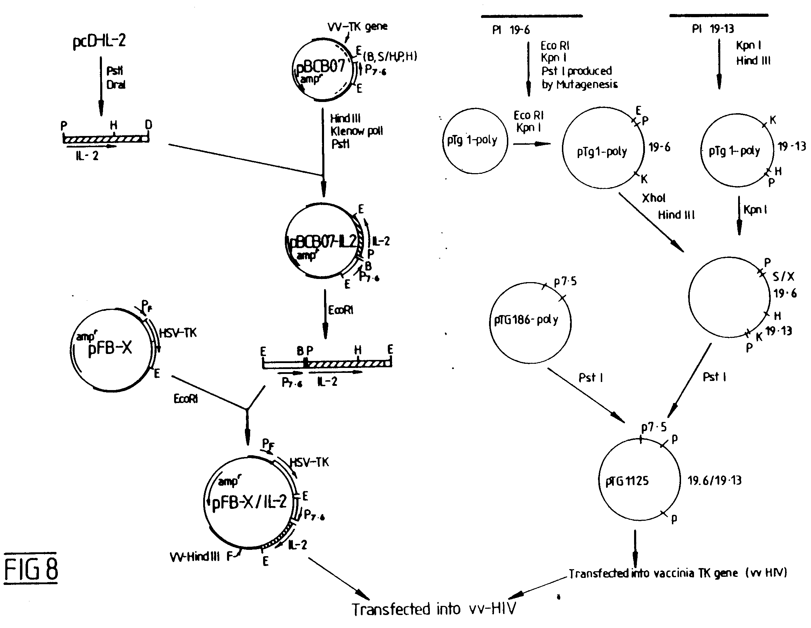

Figure 8 outlines the construction of human immunodeficiency virus IL-2 recombinant vaccinia virus in accordance with the present invention. pFB-X/IL-2 is constructed as shown using the methods described in Example 1. The construction of pTG1125 is as previously described (35).

### REFERENCES

1. Howard, M. et al. J. Exp. Med. 157, 1529-1534 (1983).
2. Baracos, V. et al. New Eng. J. Med. 308, 553-558 (1983).
3. Schmidt, J.A. et al. J. Immun. 128, 2177-2182 (1982).
4. Mizel, S.B. in Biological Response in Cancer (ed. Mihich E.) 89-119 (Plenum, New York 1982).
5. Lomedico, P.T. et al. Nature 312, 458-461 (1984).
6. Auron, P.E. et al. Proc. Natl. Acad. Sci. US 81, 7907-7911 (1984).
7. Smith, K.A. Ann. Rev. Immunol. 2, 319-333 (1984).
8. Minato, N. et al. J. Exp. Med. 154, 750 (1983).
9. Tsudo, M. et al. J. Exp. Med. 160, 612-616 (1984).
10. Merluzzi, V.J. et al. Cancer Res. 41, 850-853 (1981).
11. Wagner, H. et al. Nature 284, 278-80 (1982).
12. Vose, B.M. et al. Cancer Immuno. 13, 105-111 (1984).
13. Rosenberg, S.A. et al. Science 233, 1318-1321 (1986).
14. Yokota, T. et al. Proc. Natl. Acad. Sci. USA 82, 68-72 (1985).
15. Taniguchi, T. et al. Nature, 302, 305-307 (1983).
16. Schroder, T.W. et al. in Contemporary Topics in Molecular Immunology: The interleukins, eds Gillis, S. and Inman, F.P. (Plenum, N.Y.) Vol. 10, pp. 121-146 (1985).
17. Yokota, T. et al. Proc. Natl. Acad. Sci. USA 81, 1070-1074 (1984).
18. Sevenusar, E. Eur. J. Immunol. 17, 67-72 (1987).
19. Lee, F. et al. Proc. Natl. Acad. Sci. USA 83, 2061-2065 (1986).
20. Yokota, T. et al. Proc. Natl. Acad. Sci. USA 83, 5894-5898 (1986).
21. Gray, P.W. et al. Nature 295, 503-508 (1982).
22. Smith, G.L. et al. Nature 302, 490-495 (1983).
23. Hu, S.L. et al. Nature 320, 537-540 (1986).
24. Boyle, D.B. and Coupar, B.E.H. J. Gen. Virol. 67. 1591-1600 (1986).
25. Boyle, D.B. et al. Gene 35, 169-177 (1985).
26. Andrew, M.E. et al. Microbial Pathogenesis 1, 443-452 (1986).
27. Coupar, B.E.H. et al. J. Gen. Virol. 68, (1987), (In press).
28. Coupar, B.E.H. et al. Gene (In preparation).
29. Gillis S. et al. J. Immunol 120, 2027-2032 (1978).
30. Mosmann, J., J. Immunol. Meth. 65, 55-63 (1983).
31. Cutt, J.R. et al. J. Virol. 61, 543 (1987).
32. Wright, D.C. et al, New Eng. J. Med. 316, 673-675 (1987).
33. Kierny, M.P. et al. Biotechnology 4, 790-795 (1986).

## Claims (Claims for the following Contracting State(s): BE, CH, LI, DE, FR, GB, IT, LU, NL, SE)

1. A recombinant vaccine comprising an animal cell vector which incorporates a first nucleotide sequence capable of being expressed as all or an immunogenic part of an antigenic polypeptide, which is foreign to the host vector and is not a lymphokine, together with a second nucleotide sequence capable of being expressed as all or a lymphokine-effective part of a lymphokine other than interleukin-2 effective in enhancing the immune response to the antigenic polypeptide.

2. A recombinant vaccine according to claim 1 wherein said animal cell vector is a virus.

3. A recombinant vaccine according to claim 2, wherein said virus is a poxvirus, herpes virus or adenovirus.

4. A recombinant vaccine according to claim 3, wherein said virus is vaccinia virus.

5. A recombinant vaccine according to any one of claims 1 to 4, wherein said lymphokine is selected from the group consisting of interleukin-1, interleukin-3, interleukin-4 and γ-interferon.

6. A method for the production of a recombinant vaccine according to any one of claims 1 to 5, which comprises the step of inserting into an animal cell vector a nucleotide sequence capable of being expressed as all or a lymphokine-effective part of a lymphokine other than interleukin-2, and the further step of inserting into the animal cell vector a nucleotide sequence capable of being expressed as all or an immunogenic part of an antigenic polypeptide which is foreign to the host vector and is not a lymphokine.

7. A recombinant vaccine for use in the treatment of an immunodeficient or immunosuppressed human or animal, which comprises an animal cell vector which incorporates a first nucleotide sequence capable of being expressed as all or an immunogenic part of an antigenic polypeptide, which is not a lymphokine, together with a second nucleotide sequence capable of being expressed as all or a lymphokine-effective part of a lymphokine other than interleukin-2 effective in enhancing the immune response to the antigenic polypeptide.

8. A recombinant vaccine according to claim 7, wherein said animal cell vector is vaccinia virus.

9. A recombinant vaccine according to claim 7 or 8, wherein said lymphokine is selected from the group consisting of interleukin-1, interleukin-3, interleukin-4 and γ-interferon.

10. A recombinant vaccine according to any one of claims 1 to 5, for use in the treatment of an immunodeficient or immunosuppressed human or animal.

11. A recombinant vaccine for use in the treatment of immunodeficient or immunosuppressed individuals infected with the human immunodeficiency virus (HIV), which comprises an animal cell vector which incorporates a nucleotide sequence capable of being expressed as all or an immunogenic part of an antigenic polypeptide derived from the human immunodeficiency virus (HIV), together with a second nucleotide sequence capable of being expressed as all or a lymphokine-effective part of a lymphokine effective in enhancing the immune response of the individual to the HIV antigenic polypeptide.

12. A recombinant vaccine according to claim 11 or 12, wherein said animal cell vector is vaccinia virus.

13. A recombinant vaccine according to claim 11, wherein said lymphokine is selected from the group consisting of interleukin-1, interleukin-2, interleukin-3, interleukin-4 and γ-interferon.

14. Use of a recombinant vaccine comprising an animal cell vector which incorporates a first nucleotide sequence capable of being expressed as all or an immunogenic part of an antigenic polypeptide, which is not a lymphokine, together with a second nucleotide sequence capable of being expressed as all or a lymphokine-effective part of a lymphokine other than interleukin-2 effective in enhancing the immune response to the antigenic polypeptide, for the manufacture of a medicament for the treatment of an immunodeficient or immunosuppressed human or animal.

15. Use according to claim 14, wherein said animal cell vector is vaccinia virus.

16. Use according to claim 14, wherein said lymphokine is selected from the group consisting of interleukin-1, interleukin-3, interleukin-4 and γ-interferon.

17. Use according to claim 14 wherein the recombinant vaccine is as claimed in any one of claims 1 to 5.

## Claims (Claims for the following Contracting State(s): AT)

1. A method for the production of recombinant vaccine comprising an animal cell vector which incorporates a first nucleotide sequence capable of being expressed as all or an immunogenic part of an antigenic polypeptide, which is foreign to the host vector and is not a lymphokine, together with a second nucleotide sequence capable of being expressed as all or a lymphokine-effective part of a lymphokine other than interleukin-2 effective in enhancing the immune response to the antigenic polypeptide which method comprises the step of inserting into an animal cell vector a nucleotide sequence capable of being expressed as all or a lymphokine-effective part of a lymphokine other than interleukin-2, and the further step of inserting into the animal cell vector a nucleotide sequence capable of being expressed as all or an immunogenic part of an antigenic polypeptide which is foreign to the host vector and is not a lymphokine.

2. A method according to claim 1, wherein said animal cell vector is a virus.

3. A method according to claim 2, wherein said virus is a poxvirus, herpes virus or adenovirus.

4. A method according to claim 3, wherein said virus is vaccinia virus.

5. A method according to any one of claims 1 to 4, wherein said lymphokine is selected from the group consisting of interleukin-1, interleukin-3, interleukin-4 and γ-interferon.

6. Use of a recombinant vaccine which comprises an animal cell vector which incorporates a first nucleotide sequence capable of being expressed as all or an immunogenic part of an antigenic polypeptide, which is not a lymphokine, together with a second nucleotide sequence capable of being expressed as all or a lymphokine-effective part of a lymphokine other than interleukin-2 effective in enhancing the immune response to the antigenic polypeptide for the manufacture of a medicament for the treatment of an immunodeficient or immunosuppressed human or animal.

7. Use according to claim 6, wherein said animal cell vector is vaccinia virus.

8. Use according to claim 6 or 7, wherein said lymphokine is selected from the group consisting of interleukin-1, interleukin-3, interleukin-4 and y-interferon.

9. Use of a recombinant vaccine as defined in any one of claims 1 to 5, for the manufacture of a medicament for the treatment of an immunodeficient or immunosuppressed human or animal.

10. Use of a recombinant vaccine which comprises an animal cell vector which incorporates a nucleotide sequence capable of being expressed as all or an immunogenic part of an antigenic polypeptide derived from the human immunodeficiency virus (HIV), together with a second nucleotide sequence capable of being expressed as all or a lymphokine-effective part of a lymphokine effective in enhancing the immune response of the individual to the HIV antigenic polypeptide in the manufacture of a medicament for the treatment of immunodeficient or immunosuppressed individuals infected with the human immunodeficiency virus (HIV).

11. Use according to claim 10, wherein said animal cell vector is vaccinia virus.

12. Use according to claim 10 or 11, wherein said lymphokine is selected from the group consisting of interleukin-1, interleukin-2, interleukin-3, interleukin-4 and γ-interferon.

## Patentansprüche (Patentansprüche für folgende(n) Vertragsstaat(en): BE, CH, LI, DE, FR, GB, IT, LU, NL, SE)

1. Rekombinanter Impfstoff, der einen Vektor aus tierischen Zellen umfaßt, der eine erste Nucleotidsequenz mit der Fähigkeit, als ganzes antigenes Polypeptid oder als immunisierender Teil eines antigenen Polypeptids, das für den Wirtvektor fremd ist und kein Lymphokin darstellt, exprimiert zu werden, zusammen mit einer zweiten Nucleotidsequenz mit der Fähigkeit, als ganzes von Interleukin-2 verschiedenes Lymphokin oder als Lymphokin-wirksamer Teil eines von Interleukin-2 verschiedenen Lymphokins, das eine Verstärkung der Immunantwort auf das antigene Polypeptid bewirkt, exprimiert zu werden, beinhaltet.

2. Rekombinanter Impfstoff nach Anspruch 1, wobei der Vektor aus tierischen Zellen ein Virus ist.

3. Rekombinanter Impfstoff nach Anspruch 2, wobei das Virus ein Pockenvirus, Herpesvirus oder Adenovirus ist.

4. Rekombinanter Impfstoff nach Anspruch 3, wobei das Virus ein Kuhpockenvirus ist.

5. Rekombinanter Impfstoff nach einem der Ansprüche 1 bis 4, wobei das Lymphokin unter Interleukin-1, Interleukin-3, Interleukin-4 und γ-Interferon ausgewählt ist.

6. Verfahren zur Herstellung eines rekombinanten Impfstoffs nach einem der Ansprüche 1 bis 5 durch Insertieren einer Nucleotidsequenz mit der Fähigkeit, als ganzes von Interleukin-2 verschiedenes Lymphokin oder als Lymphokin-wirksamer Teil eines von Interleukin-2 verschiedenen Lymphokins exprimiert zu werden, in einen Vektor aus tierischen Zellen und des weiteren Insertieren einer Nucleotidsequenz mit der Fähigkeit, als ganzes antigenes Polypeptid oder als immunisierender Teil eines antigenen Polypeptids, das für den Wirtvektor fremd ist und kein Lymphokin darstellt, exprimiert zu werden, in den Vektor aus tierischen Zellen.

7. Rekombinanter Impfstoff zur Verwendung bei der Behandlung von Menschen oder Tieren mit Immunodefizienz oder Immunosuppression, der einen Vektor aus tierischen Zellen umfaßt, der eine erste Nucleotidsequenz mit der Fähigkeit, als ganzes antigenes Polypeptid oder als immunisierender Teil eines antigenen Polypeptids, das für den Wirtvektor fremd ist und kein Lymphokin darstellt, exprimiert zu werden, zusammen mit einer zweiten Nucleotidsequenz mit der Fähigkeit, als ganzes von Interleukin-2 verschiedenes Lymphokin oder als Lymphokinwirksamer Teil eines von Interleukin-2 verschiedenen Lymphokins, das eine Verstärkung der Immunantwort auf das antigene Polypeptid bewirkt, exprimiert zu werden, beinhaltet.

8. Rekombinanter Impfstoff nach Anspruch 7, wobei der Vektor aus tierischen Zellen ein Kuhpockenvirus ist.

9. Rekombinanter Impfstoff nach Anspruch 7 oder 8, wobei das Lymphokin unter Interleukin-1, Interleukin-3, Interleukin-4 und γ-Interferon ausgewählt ist.

10. Rekombinanter Impfstoff nach einem der Ansprüche 1 bis 5 zur Verwendung bei der Behandlung von Menschen oder Tieren mit Immunodefizienz oder Immunosuppression.

11. Rekombinanter Impfstoff zur Verwendung bei der Behandlung von eine Immunodefizienz oder Immunosuppression aufweisenden Individuen, die mit dem Humanimmunodefizienzvirus (HIV) infiziert sind, der einen Vektor aus tierischen Zellen umfaßt, der eine erste Nucleotidsequenz mit der Fähigkeit, als ganzes antigenes Polypeptid oder als immunisierender Teil eines antigenen Polypeptids, das vom Humanimmunodefizienzvirus (HIV) herrührt, exprimiert zu werden, zusammen mit einer zweiten Nucleotidsequenz mit der Fähigkeit, als ganzes von Interleukin-2 verschiedenes Lymphokin oder als Lymphokinwirksamer Teil eines von Interleukin-2 verschiedenen Lymphokins, das eine Verstärkung der Immunantwort des Individuums auf das antigene HIV-Polypeptid bewirkt, exprimiert zu werden, beinhaltet.

12. Rekombinanter Impfstoff nach Anspruch 11, wobei der Vektor aus tierischen Zellen ein Kuhpockenvirus ist.

13. Rekombinanter Impfstoff nach Anspruch 11 oder 12, wobei das Interleukin unter Interleukin-1, Interleukin-2, Interleukin-3, Interleukin-4 und γ-Interferon ausgewählt ist.

14. Verwendung eines rekombinanten Impfstoffs, der einen Vektor aus tierischen Zellen umfaßt, der eine erste Nucleotidsequenz mit der Fähigkeit, als ganzes antigenes Polypeptid oder als immunisierender Teil eines antigenen Polypeptids, das kein Lymphokin darstellt, exprimiert zu werden, zusammen mit einer zweiten Nucleotidsequenz mit der Fähigkeit, als ganzes von Interleukin-2 verschiedenes Lymphokin oder als Lymphokin-wirksamer Teil eines von Interleukin-2 verschiedenen Lymphokins, das eine Verstärkung der Immunantwort auf das antigene Polypeptid bewirkt, exprimiert zu werden, beinhaltet, zur Herstellung eines Medikaments zur Behandlung eines Menschen oder Tiers mit Immunodefizienz oder Immunosuppression.

15. Verwendung nach Anspruch 14, wobei der Vektor aus tierischen Zellen ein Kuhpockenvirus ist.

16. Verwendung nach Anspruch 14, wobei das Lymphokin unter Interleukin-1, Interleukin-3, Interleukin-4 und γ-Interferon ausgewählt ist.

17. Verwendung nach Anspruch 14, wobei der rekombinante Impfstoff ein solcher ist, wie er in einem der Ansprüche 1 bis 5 beansprucht wird.

## Patentansprüche (Patentansprüche für folgende(n) Vertragsstaat(en): AT)

1. Verfahren zur Herstellung eines rekombinanten Impfstoffs mit einem Vektor aus tierischen Zellen, der eine erste Nucleotidsequenz mit der Fähigkeit, als ganzes antigenes Polypeptid oder als immunisierender Teil eines antigenen Polypeptids, das für den Wirtvektor fremd ist und kein Lymphokin darstellt, exprimiert zu werden, zusammen mit einer zweiten Nucleotidsequenz mit der Fähigkeit, als ganzes von Interleukin-2 verschiedenes Lymphokin oder als Lymphokinwirksamer Teil eines von Interleukin-2 verschiedenen Lymphokins, das eine Verstärkung der Immunantwort auf das antigene Polypeptid bewirkt, exprimiert zu werden, beinhaltet, durch Insertieren einer Nucleotidsequenz mit der Fähigkeit, als ganzes von Interleukin-2 verschiedenes Lymphokin oder als Lymphokin-wirksamer Teil eines von Interleukin-2 verschiedenen Lymphokins exprimiert zu werden, in einen Vektor aus tierischen Zellen und des weiteren Insertieren einer Nucleotidsequenz mit der Fähigkeit, als ganzes antigenes Polypeptid oder als immunisierender Teil eines antigenen Polypeptids, das für den Wirtvektor fremd ist und kein Lymphokin darstellt, exprimiert zu werden, in den Vektor aus tierischen Zellen.

2. Verfahren nach Anspruch 1, wobei der Vektor aus tierischen Zellen ein Virus ist.

3. Verfahren nach Anspruch 2, wobei das Virus ein Pockenvirus, Herpesvirus oder Adenovirus ist.

4. Verfahren nach Anspruch 3, wobei das Virus ein Kuhpockenvirus ist.

5. Verfahren nach einem der Ansprüche 1 bis 4, wobei das Lymphokin unter Interleukin-1, Interleukin-3, Interleukin-4 und γ-Interferon ausgewählt ist.

6. Verwendung eines rekombinanten Impfstoffs mit einem Vektor aus tierischen Zellen, der eine erste Nucleotidsequenz mit der Fähigkeit, als ganzes antigenes Polypeptid oder als immunisierender Teil eines antigenen Polypeptids, das für den Wirtvektor fremd ist und kein Lymphokin darstellt, exprimiert zu werden, zusammen mit einer zweiten Nucleotidsequenz mit der Fähigkeit, als ganzes von Interleukin-2 verschiedenes Lymphokin oder als Lymphokin-wirksamer Teil eines von Interleukin-2 verschiedenen Lymphokins, das eine Verstärkung der Immunantwort auf das antigene Polypeptid bewirkt, exprimiert zu werden, beinhaltet, bei der Behandlung von Menschen oder Tieren mit Immunodefizienz oder Immunosuppression.

7. Verwendung nach Anspruch 6, wobei der Vektor aus tierischen Zellen ein Kuhpockenvirus ist.

8. Verwendung nach Anspruch 6 oder 7, wobei das Lymphokin unter Interleukin-1, Interleukin-3 Interleukin-4 und y-Interferon ausgewählt ist.

9. Verwendung eines rekombinanten Impfstoffs nach einem der Ansprüche 1 bis 5 zur Herstellung eines Medikaments zur Behandlung eines Menschen oder Tiers mit Immunodefizienz oder Immunosuppression.

10. Verwendung eines rekombinanten Impfstoffs zur Herstellung eines Medikaments mit einem Vektor aus tierischen Zellen, der eine erste Nucleotidsequenz mit der Fähigkeit, als ganzes antigenes Polypeptid oder als immunisierender Teil eines antigenen Polypeptids, das vom Humanimmunodefizienzvirus (HIV) herrührt, exprimiert zu werden, zusammen mit einer zweiten Nucleotidsequenz mit der Fähigkeit, als ganzes von Interleukin-2 verschiedenes Lymphokin oder als Lymphokinwirksamer Teil eines von Interleukin-2 verschiedenen Lymphokins, das eine Verstärkung der Immunantwort des Individuums auf das antigene HIV-Polypeptid bewirkt, exprimiert zu werden, beinhaltet, zur Behandlung von eine Immunodefizienz oder Immunosuppression aufweisenden Individuen, die mit dem humanimmunodefizienzvirus (HIV) infiziert sind.

11. Verwendung nach Anspruch 10, wobei der Vektor aus tierischen Zellen ein Kuhpockenvirus ist.

12. Verwendung nach Anspruch 10 oder 11, wobei das Interleukin unter Interleukin 1-, Interleukin-2, Interleukin-3, Interleukin-4 und γ-Interferon ausgewählt ist.

## Revendications (Revendications pour l'(les) Etat(s) contractant(s) suivant(s): BE, CH, LI, DE, FR, GB, IT, LU, NL, SE)

1. Vaccin recombinant comprenant un vecteur cellulaire animal qui incorpore une première séquence nucléotidique capable d'être exprimée sous la forme de la totalité ou d'une partie immunogène d'un polypeptide antigénique, qui est étranger au vecteur hôte et n'est pas une lymphokine, avec une seconde séquence nucléotidique capable d'être exprimée sous la forme de la totalité ou d'une partie à action de lymphokine d'une lymphokine autre que l'interleukine-2 efficace pour accroître la réponse immune au polypeptide antigénique.

2. Vaccin recombinant selon la revendication 1 dans lequel ledit vecteur cellulaire animal est un virus.

3. Vaccin recombinant selon la revendication 2, dans lequel ledit virus est un pox virus, un herpes virus ou un adénovirus.

4. Vaccin recombinant selon la revendication 3, dans lequel ledit virus est le virus de la vaccine.

5. Vaccin recombinant selon l'une quelconque des revendications 1 à 4, dans lequel ladite lymphokine est choisie dans le groupe constitué par l'interleukine-1, l'interleukine-3, l'interleukine-4 et l'interféron γ.

6. Procédé de production d'un vaccin recombinant selon l'une quelconque des revendications 1 à 5, qui comprend l'étape d'insertion, dans un vecteur cellulaire animal, d'une séquence nucléotidique capable d'être exprimée sous la forme de la totalité ou d'une partie à efficacité de lymphokine d'une lymphokine autre que l'interleukine-2, et l'étape ultérieure d'insertion, dans un vecteur cellulaire animal, d'une séquence nucléotidique capable d'être exprimée sous la forme de la totalité ou d'une partie immunogène d'un polypeptide antigénique qui est étranger au vecteur hôte et qui n'est pas une lymphokine.

7. Vaccin recombinant pour utilisation dans un traitement d'un être humain ou d'un animal immunodéficient ou ayant subi une immunosuppression, qui comprend un vecteur cellulaire animal incorporant une première séquence nucléotidique capable d'être exprimée sous la forme de la totalité ou d'une partie immunogène d'un polypeptide antigénique, qui n'est pas une lymphokine, avec une seconde séquence nucléotidique capable d'être exprimée sous la forme de la totalité ou d'une partie à efficacité de lymphokine autre que l'interleukine-2 efficace pour accroître la réponse immune au polypeptide antigénique.

8. Vaccin recombinant selon la revendication 7, dans lequel ledit vecteur cellulaire animal est le virus de la vaccine.

9. Vaccin recombinant selon la revendication 7 ou 8, dans lequel ladite lymphokine est choisie dans le groupe constitué par l'interleukine-1, l'interleukine-3, l'interleukine-4 et l'interféron γ.

10. Vaccin recombinant selon l'une quelconque des revendications 1 à 5, aux fins d'utilisation dans le traitement d'un être humain ou d'un animal immunodéficient ou ayant subi une immunosuppression.

11. Vaccin recombinant pour utilisation dans le traitement des individus immunodéficients ou ayant subi une immunosuppression infectés par le virus d'immunodéficience humaine (HIV), qui comprend un vecteur cellulaire animal incorporant une séquence nucléotidique capable d'être exprimée sous la forme de la totalité ou d'une partie immunogène d'un polypeptide antigénique dérivé du virus de l'immunodéficience humaine (HIV), avec une seconde séquence nucléotidique capable d'être exprimée sous la forme de la totalité ou d'une partie à action de lymphokine d'une lymphokine efficace pour accroître la réponse immune de l'individu au polypeptide antigénique d'HIV.

12. Vaccin recombinant selon la revendication 11 dans lequel ledit vecteur cellulaire animal est le virus de la vaccine.

13. Vaccin recombinant selon la revendication 11 ou 12, dans lequel ladite lymphokine est choisie dans le groupe constitué par l'interleukine-1, l'interleukine-2, l'interleukine-3, l'interleukine-4 et l'interféron γ.

14. Application d'un vaccin recombinant comprenant un vecteur cellulaire animal qui incorpore une première séquence nucléotidique capable d'être exprimée sous la forme de la totalité ou d'une partie immunogène d'un polypeptide antigénique, qui n'est pas une lymphokine, avec une seconde séquence nucléotidique capable d'être exprimée sous la forme de la totalité ou d'une partie à action de lymphokine d'une lymphokine autre que l'interleukine-2 efficace pour accroître la réponse immune au polypeptide antigénique, à la préparation d'un médicament pour le traitement d'un être humain ou d'un animal immunodéficient ou ayant subi une immunosuppression.

15. Application selon la revendication 14, dans laquelle ledit vecteur cellulaire animal est le virus de la vaccine.

16. Application selon la revendication 14, dans laquelle ladite lymphokine est choisie dans le groupe constitué par l'interleukine-1, l'interleukine-3, l'interleukine-4 et l'interféron γ.

17. Application selon la revendication 14 dans laquelle le vaccin recombinant est tel que revendiqué dans l'une quelconque des revendications 1 à 5.

## Revendications (Revendications pour l'(les) Etat(s) contractant(s) suivant(s): AT)

1. Procédé de production d'un vaccin recombinant comprenant un vecteur cellulaire animal qui incorpore une première séquence nucléotidique capable d'être exprimée sous la forme de la totalité ou d'une partie immunogène d'un polypeptide antigénique, qui est étranger au vecteur hôte et n'est pas une lymphokine, avec une seconde séquence nucléotidique capable d'être exprimée sous la forme de la totalité ou d'une partie à action de lymphokine d'une lymphokine autre que l'interleukine-2 efficace pour accroître la réponse immune au polypeptide antigénique, ce procédé comprenant l'étape d'insertion dans un vecteur cellulaire animal d'une séquence nucléotidique capable d'être exprimée sous la forme de la totalité ou d'une partie à action de lymphokine d'une lymphokine autre que l'interleukine-2, et l'étape ultérieure d'insertion dans le vecteur cellulaire animal d'une séquence nucléotidique capable d'être exprimée sous la forme de la totalité ou d'une partie immunogène d'un polypeptide antigénique qui est étrangère au vecteur hôte et n'est pas une lymphokine.

2. Procédé selon la revendication 1 dans lequel ledit vecteur cellulaire animal est un virus.

3. Procédé selon la revendication 2, dans lequel ledit virus est un pox virus, un herpes virus ou un adénovirus.

4. Procédé selon la revendication 3, dans lequel ledit virus est le virus de la vaccine.

5. Procédé selon l'une quelconque des revendications 1 à 4, dans lequel ladite lymphokine est choisie dans le groupe constitué par l'interleukine-1, l'interleukine-3, l'interleukine-4 et l'interféron γ.

6. Application d'un vaccin recombinant qui comprend un vecteur cellulaire animal incorporant une première séquence nucléotidique capable d'être exprimée sous la forme de la totalité ou d'une partie immunogène d'un polypeptide antigénique qui n'est pas une lymphokine, avec une seconde séquence nucléotidique capable d'être exprimée sous la forme de la totalité ou d'une partie à action de lymphokine d'une lymphokine autre que l'interleukine-2 efficace pour accroître la réponse immunitaire au polypeptide antigénique pour la production d'un médicament pour le traitement d'un être humain ou d'un animal immunodéficient ou ayant subi une immunosuppression.

7. Application selon la revendication 6, dans laquelle ledit vecteur cellulaire animal est le virus de la vaccine.

8. Application selon la revendication 6 ou 7, dans laquelle ladite lymphokine est choisie dans le groupe constitué par l'interleukine-1, l'interleukine-3, l'interleukine-4 et l'interféron γ.

9. Application d'un vaccin recombinant tel que défini selon l'une quelconque des revendications 1 à 5 à la production d'un médicament pour le traitement d'un être humain ou d'un animal immunodéficient ou ayant subi une immunosuppression.

10. Application d'un vaccin recombinant qui comprend un vecteur cellulaire animal incorporant une séquence nucléotidique capable d'être exprimée sous la forme de la totalité ou d'une partie immunogène d'un polypeptide antigénique dérivé du virus de l'immunodéficience humaine (HIV), avec une seconde séquence nucléotidique capable d'être exprimée sous la forme de la totalité ou d'une partie à action de lymphokine d'une lymphokine efficace pour accroître la réponse immune de l'individu au polypeptide antigénique d'HIV à la production d'un médicament pour le traitement des individus immunodéficients ou ayant subi une immunosuppression infectés par le virus de l'immunodéficience humaine.

11. Application selon la revendication 10, dans laquelle ledit vecteur cellulaire animal est le virus de la vaccine.

12. Application selon la revendication 10 ou 11, dans laquelle ladite lymphokine est choisie dans le groupe constitué par l'interleukine-1, l'interleukine-2, l'interleukine-3, l'interleukine-4 et l'interféron γ.
